# EUROPÄISCHE PATENTANMELDUNG

(11) **EP 3 421 083 A1**
(43) Veröffentlichungstag der Anmeldung: **02.01.2019**
(21) Anmeldenummer: 17179014.0
(22) Anmeldetag: 30.06.2017
(51) Int. Cl.: A61N 1/375, A61N 1/372, H04B 10/25, H04B 10/80

(54) **DURCHFÜHRUNG EINES MEDIZINELEKTRONISCHEN GERÄTES UND MEDIZINELEKTRONISCHES GERÄT**

(71) Anmelder: BIOTRONIK SE & Co. KG, 12359 Berlin (DE)
(72) Erfinder: Henschel, Martin, 13125 Berlin (DE)
(74) Vertreter: Keck, Hans-Georg

(57) **Zusammenfassung**

Durchführung eines, insbesondere implantierbaren, medizinelektronischen Gerätes, welches ein Gerätegehäuse aufweist, in dem elektronische und/oder elektrische Funktionseinheiten untergebracht sind und das eine mit der Durchführung verschlossene Gehäuseöffnung hat, wobei die Durchführung einen Isolierkörper, einen den Isolierkörper umfassenden und an der Gehäuseöffnung halternden Durchführungs-Flansch und eine Mehrzahl von den Isolierkörper durchstoßenden Anschlusselementen zum externen Anschluss mindestens einer Funktionseinheit des Gerätes aufweist, wobei mindestens ein Anschlusselement ein hermetisch dicht in den Isolierkörper oder direkt in den Durchführungs-Flansch eingefügtes Licht- oder Infrarotfenster aufweist.

## Beschreibung

Die Erfindung betrifft eine Durchführung eines implantierbaren medizinelektronischen Gerätes sowie auch ein derartiges Gerät. Dieses umfasst typischerweise ein Gerätegehäuse, in dem elektronische und elektrische Funktionseinheiten untergebracht sind. Eine derartige Durchführung umfasst einen Isolierkörper, insbesondere aus Keramik oder Glas, einen den Isolierkörper umfassenden Durchführungs-Flansch und mindestens ein den Isolierkörper durchstoßendendes Anschlusselement zum externen Anschluss eines elektrischen oder elektronischen Bauelements des Geräts.

Implantierbare Geräte der oben bezeichneten Art sind insbesondere als Herzschrittmacher, implantierbare Kardioverter (speziell Defibrillatoren) oder auch als Cochlearimplantate seit langem in massenhaftem Einsatz. Es kann sich hierbei aber auch um eine weniger komplexe Vorrichtung, wie etwa eine Elektroden- oder Sensorleitung handeln. Insbesondere kann es ich auch um Neurostimulatoren oder Retinaimplantate oder ähnliche Geräte zur Beeinflussung der Sinneswahrnehmung bzw. Gehirn- und Nerventätigkeit handeln.

Die meisten praktisch bedeutsamen implantierbaren medizinelektronischen Geräte sind dazu vorgesehen, über geeignet platzierte Elektroden elektrische Impulse an reizbares Körpergewebe abzugeben oder elektrische Signale des Körpers zu messen. Um diese Funktion auszuführen, sind im Gehäuse des Gerätes elektronische/elektrische Funktionseinheiten zur Verarbeitung der Impulse und/oder zur geeigneten Steuerung der Reizerzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind.

Hierfür muss eine elektrische Verbindung zwischen den im Gehäuseinneren angeordneten elektrischen und/oder elektronischen Bauteilen und der jeweiligen Elektrodenleitung bzw. den extern angeordneten Elektroden und/oder Sensoren hergestellt werden. Diese elektrische Verbindung wird in der Regel mittels einer Durchführung und/oder einem sogenannten Header realisiert.

Eine derartige Durchführung sorgt dabei für mindestens eine elektrische Verbindung zwischen dem Inneren des Gehäuses und dem Äußeren und schließt gleichzeitig das Gehäuse des Implantats hermetisch ab. Der über der Durchführung befestigte Header führt die elektrische Verbindung der Durchführung weiter zu einer Kontaktstelle und dient zum Einstecken der mindestens einen Elektrodenleitung in eine entsprechende, meist genormte Buchse. An den Kontaktstellen der Buchse wird so ein elektrischer Kontakt zwischen dem Implantat und dem Anschlussstück der Elektrodenleitung hergestellt. Eine Durchführung und ein Header können auch in einem einzigen Bauteil realisiert sein. Auch in diesem Fall wird ein solches kombiniertes Bauteil im Folgenden allgemein als Durchführung bezeichnet.

Aus der US 5,902,326 ist das Vorsehen eines optischen Fensters in einem implantierbaren medizinelektronischen Gerät zur Übertragung optischer Energie bekannt, wobei das Fenster auch linsenartig ausgeführt sein kann. Es wird beschrieben, dass das Fenster in der Gehäusehülle angeordnet ist, und seine Funktion wird nicht näher beschrieben. Es ist auch eine Anwendung des detailliert beschriebenen Fensteraufbaus zur Isolierung der Stifte einer Durchführung eines elektromedizinischen Gerätes erwähnt.

Aus der US 7,349,618 B2 ist es bekannt, in eine Durchführung eines implantierbaren medizinelektronischen Gerätes einen Lichtleiterabschnitt einzusetzen, mit dem die Signale von außerhalb des Gerätes angeordneten Sensoren für physiologische Parameter über eine optische Übertragungsstrecke in das Gerät übertragen werden.

Bei den meisten Geräten der oben beschriebenen Art müssen mehrere, teilweise sehr viele, Signalverbindungen zwischen Funktionseinheiten im Inneren des Gerätes und externen Signalquellen oder -Empfängern hergestellt werden. Entsprechend viele Kontaktelemente sind in bekannten Durchführungen enthalten, und diese müssen jeweils zuverlässig hermetisch dicht ausgeführt werden. Die Realisierung von hermetisch abgedichteten elektrischen Anschlusselementen ist kostenaufwändig. Zudem wird auf beiden Seiten der Durchführung für den Anschluss der jeweiligen einzelnen Kontaktelemente Bauraum benötigt, was bei einer relativ großen Anzahl von Signalverbindungen zu Lasten der erwünschten Kompaktheit des Gerätes geht. Speziell bei neuartigen Geräten mit komplexen Stimulations- bzw. Sensorfunktionen, wie etwa Neurostimulatoren oder Retinaimplantaten, ergeben sich hieraus widersprüchliche Anforderungen, die nur schwer zu erfüllen sind.

Der Erfindung liegt daher die Aufgabe zugrunde, eine verbesserte Durchführung anzugeben, die insbesondere grundsätzlich die Realisierung einer relativ großen Anzahl von Signalverbindungen zwischen dem Inneren eines elektromedizinischen Gerätes und dem Außenraum mit verringertem Platzbedarf und verringertem konstruktiven und Herstellungsaufwand und somit reduzierten Kosten ermöglicht.

Diese Aufgabe wird durch eine Durchführung mit den Merkmalen des Anspruchs 1 gelöst. Zweckmäßige Fortbildungen des Erfindungsgedankens sind Gegenstand der abhängigen Ansprüche. Weiterhin wird ein neuartiges elektromedizinisches Gerät mit den Merkmalen des Anspruchs 7 bzw. Anspruchs 9 bereitgestellt, und dessen Fortbildungen sind Gegenstand der von Anspruch 7 bzw. 9 abhängigen Ansprüche.

In einer Ausführung ist das Licht- oder Infrarotfenster ein in den Isolierkörper oder direkt in den Durchführungs-Flansch eingeschmolzenes oder eingeklebtes Glasfenster. In einer alternativen Ausführung ist das Licht- oder Infrarotfenster ein in den Isolierkörper oder direkt in den Durchführungs-Flansch eingespritztes, eingepresstes oder eingeklebtes Kunststofffenster.

Im Zusammenhang mit der Erfindung wird unter einem Licht- oder Infrarotfenster jedoch allgemein ein Element verstanden, welches für Licht im sichtbaren Bereich oder Infrarotstrahlung, insbesondere in dem für Signalübertragungen üblicherweise genutzten Bereich, im Wesentlichen durchlässig ist. Die Ausführung der Erfindung ist daher nicht auf Fenster aus Glas oder transparentem Kunststoff beschränkt; insbesondere für IR-Strahlung kommen weitere Materialien in Betracht, deren IR-Durchlässigkeit dem Fachmann bekannt ist und die er daher gemäß den Anforderungen der konkreten Anwendung auswählen wird.

In zweckmäßigen Ausgestaltungen der Erfindung ist das Licht- oder Infrarotfenster als Kreisscheibe oder als einen zylindrischen Anschlussstift umgebender Kreisring konfiguriert. Diese Ausführungen sind an den herkömmlichen Aufbau von Durchführungen mit typischerweise kreisförmigen Bohrungen für die Anschlusselemente angepasst; es sind jedoch grundsätzlich andere geometrische Konfigurationen möglich, wie etwa rechteckige oder solche in Art eines Langloches.

Üblicherweise besteht der Isolierkörper aus einer Keramik, und die Technologien zu seiner Herstellung und zum hermetisch dichten Einfügen von Glas- oder Kunststoffelementen in einen solchen Keramikkörper sind grundsätzlich verfügbar und dem Fachmann bekannt. Daher kann von einer genaueren Beschreibung hier abgesehen werden. Die Erfindung ist jedoch auch bei Durchführungen mit einem Isolierkörper aus anderem Material einsetzbar, der etwa aus Glas oder Kunststoff besteht bzw. Glas- oder Kunststoffteile aufweist.

Obgleich aus derzeitiger Sicht Ausführungen der Erfindung als bevorzugt erscheinen, bei denen ein Licht- oder Infrarotfenster beispielsweise aus Glas oder Kunststoff in einen Keramik-Isolierkörper eingeschmolzen oder eingespritzt ist, kann grundsätzlich der Isolierkörper mindestens teilweise auch durch das Licht- oder Infrarotfenster selbst gebildet sein. Dieses hat dann mindestens über einen Teilbereich seines Umfangs direkten Kontakt mit dem Durchführungs-Flansch. Weiterhin ist es grundsätzlich möglich, das Licht- oder Infrarotfenster auch direkt in das Gerätegehäuse einzubinden, also nicht in eine spezielle Durchführung oder deren Isolierkörper bzw. Flansch.

Bei dem vorgeschlagenen medizinelektronischen Gerät handelt es sich insbesondere um ein implantierbares Gerät und noch spezieller um einen Neurostimulator, ein Cochlearimplantat oder Retinaimplantat oder ein ähnlich komplexes Gerät mit zahlreichen internexternen Signalverbindungen. Jedoch ist die Anwendung der Erfindung nicht auf solche komplexen Geräte beschränkt, sondern auch bei Geräten mit einer geringeren Anzahl an Signalverbindungen vorteilhaft möglich, wie etwa bei Herzschrittmachern oder Kardiovertern.

In Ausführungen des Gerätes weist dieses ein Kopfteil auf, wobei die Durchführung das Gerätegehäuse vom Kopfteil trennt. Die Erfindung ist jedoch auch bei Geräten einsetzbar, bei denen die Durchführung unmittelbar Teil der Außenhülle des Gerätes ist, bei denen also ein Kopfteil im eigentlichen Sinne nicht vorhanden ist.

In zweckmäßigen Ausführungen der Erfindung ist dem oder mindestens einem Licht- oder Infrarotfenster im Gerätegehäuse und/oder im Kopfteil mindestens ein Lichtleiterabschnitt zugeordnet, über den eine optische Signalübertragung durch das Fenster hindurch realisiert wird. In einer Ausgestaltung ist ein freies Ende des Lichtleiters oder der Lichtleiter direkt an eine Oberfläche des Licht- oder Infrarotfensters angefügt.

Bei Geräten mit dem oben erwähnten komplexen Aufbau ist insbesondere dem oder mindestens einem Licht- oder Infrarotfenster eine Mehrzahl von Lichtleiterabschnitten zugeordnet, derart, dass durch das Fenster hindurch eine mehrkanalige optische Signalübertragung realisiert wird. Auch unabhängig vom Vorsehen mehrerer dem Fenster zugeordneter Lichtleiterabschnitte kann mit bekannten Mitteln der optischen Signalübertragung (wie Wellenlängen- oder Phasen-Multiplexing) eine mehrkanalige Signalübertragung durch das Fenster realisiert werden.

In den zuletzt erwähnten Ausführungen der Erfindung kommen deren Vorteile besonders deutlich zum Tragen: Das optische Signalübertragungs-Fenster ermöglicht die Übertragung einer Vielzahl verschiedener Signale über ein einziges Element der Durchführung, statt - wie beim Stand der Technik über eine Vielzahl separater und einzeln abzudichtender Anschlusselemente. Dies kann, je nach Anzahl der eingesparten separaten Anschlusselemente, zu erheblichen Kostensenkungen für die Durchführung führen und bei geeigneter Ausgestaltung der Signalzu- und -ableitung auch den auf einer Seite oder beiden Seiten der Durchführung benötigten Bauraum für die Anschlüsse erheblich verringern. Es kann auch zur spürbaren Verringerung von Ausschuss und Zuverlässigkeitsproblemen gegenüber bekannten Durchführungen mit einer Vielzahl einzelner Anschlusselemente führen.

In weiteren Ausführungen des erfindungsgemäßen Gerätes ist dem oder mindestens einem Licht- oder Infrarotfenster mindestens eine LED oder ein Halbleiterlaserelement zugeordnet, über die/das eine optische Signalübertragung durch das Fenster hindurch realisiert wird. Je nach konkreter Anwendung können auch jeweils mehrere LEDs oder Halbleiterlaserelemente zur Realisierung mehrerer Signalübertragungen dem Fenster zugeordnet sein, oder es kann in der Durchführung auch mehr als ein einzelnes Fenster angeordnet sein.

In einer weiteren Ausführung der Erfindung ist in der Durchführung ein einen zylindrischen Anschlussstift umgebendes kreisringförmiges Licht- oder Infrarotfenster vorgesehen und der vom Fenster umgebene Anschlussstift ist mit einem Versorgungsstromleiter zur Energieversorgung mindestens einer Funktionseinheit des Gerätes verbunden. Hierbei wird also durch die Anschlussstift/Fenster-Kombination eine kombinierte Energieversorgung und Signalübertragung praktisch über eine einzelne abzudichtende Öffnung der Durchführung realisiert. Auch dies führt zu Vorteilen der weiter oben beschriebenen Art.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine schematische, teilweise geschnittene Darstellung eines implantierbaren medizinelektronischen Geräts,
- Fig. 2 und 2a: eine perspektivische Darstellung einer Durchführung sowie eine teilweise geschnittene Detailansicht gemäß einer Ausführungsform der Erfindung,
- Fig. 3 und 3a: eine perspektivische Darstellung einer Durchführung sowie eine teilweise geschnittene Detailansicht gemäß einer Ausführungsform der Erfindung,
- Fig. 4 und 4a: eine perspektivische Darstellung einer Durchführung sowie eine teilweise geschnittene Detailansicht gemäß einer Ausführungsform der Erfindung,
- Fig. 5 und 5a: eine perspektivische Darstellung einer Durchführung sowie eine teilweise geschnittene Detailansicht gemäß einer Ausführungsform der Erfindung,
- Fig. 6 und 6a: eine perspektivische Darstellung einer Durchführung sowie eine teilweise geschnittene Detailansicht gemäß einer Ausführungsform der Erfindung,
- Fig. 7 und 7a: eine perspektivische Darstellung einer Durchführung sowie eine teilweise geschnittene Detailansicht gemäß einer Ausführungsform der Erfindung,
- Fig. 8 und 8a: eine teilweise geschnittene perspektivische Darstellung eines implantierbaren medizinelektronischen Gerätes mit Kopfteil (Header) sowie eine Detailansicht hierzu gemäß einer weiteren Ausführungsform der Erfindung,
- Fig. 9: eine teilweise geschnittene perspektivische Prinzipskizze einer Durchführung gemäß einer weiteren Ausführung der Erfindung, und
- Fig. 10: eine teilweise geschnittene perspektivische Prinzipskizze einer Durchführung gemäß einer weiteren Ausführung der Erfindung.

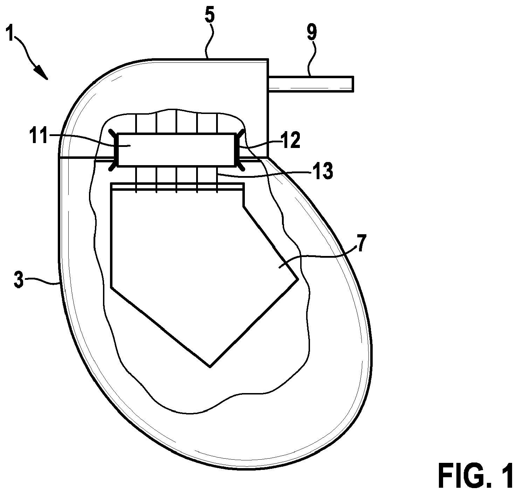

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11, die in einem Durchführungs-Flansch 12 gehaltert ist, umfasst eine Mehrzahl von Anschlusselementen 13 zum externen Anschluss der Leiterplatte 7.

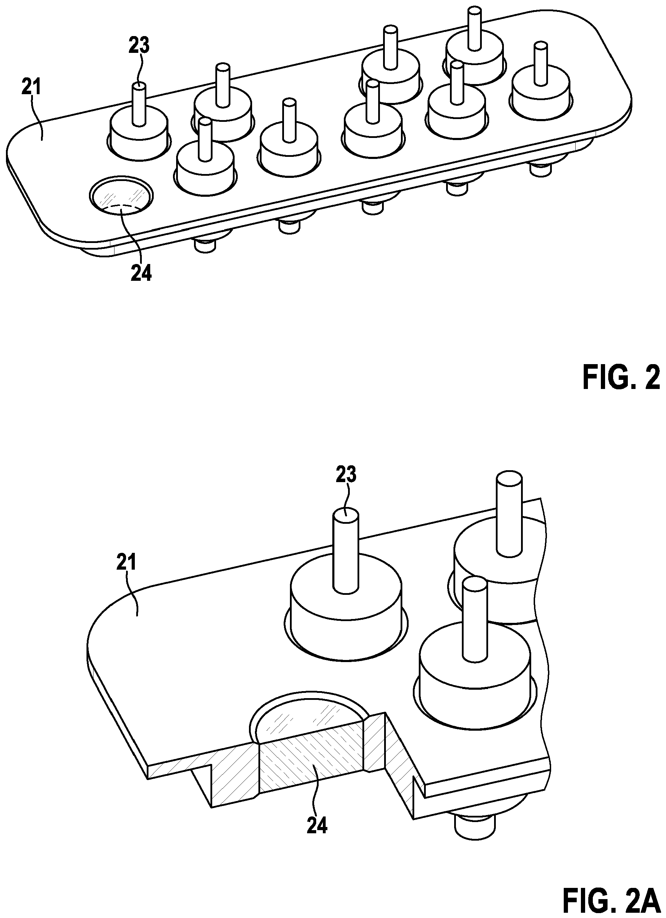

In den Figuren 2 bis 8a sind Teile, die bestimmten in Fig. 1 gezeigten Teilen entsprechen, mit an diese angelehnten Bezugsziffern bezeichnet.

Fig. 2 und 2a zeigen eine erfindungsgemäße Durchführung 21, in der neben einer Mehrzahl von Anschlusselementen (Kontaktstiften) 23 in konventioneller Ausführung eine Glasscheibe 24 vorgesehen ist, die in der Position, die normalerweise ein weiterer Anschlussstift einnehmen würde, in das Keramikmaterial der Durchführung mittels des GMTS (Glass-to-Metal-Seal)-Verfahrens eingebracht wurde. Das Verfahren zur Einbringung einer solchen Glasscheibe als Lichtfenster ist dem Fachmann an sich vertraut und bedarf daher hier keiner genaueren Beschreibung.

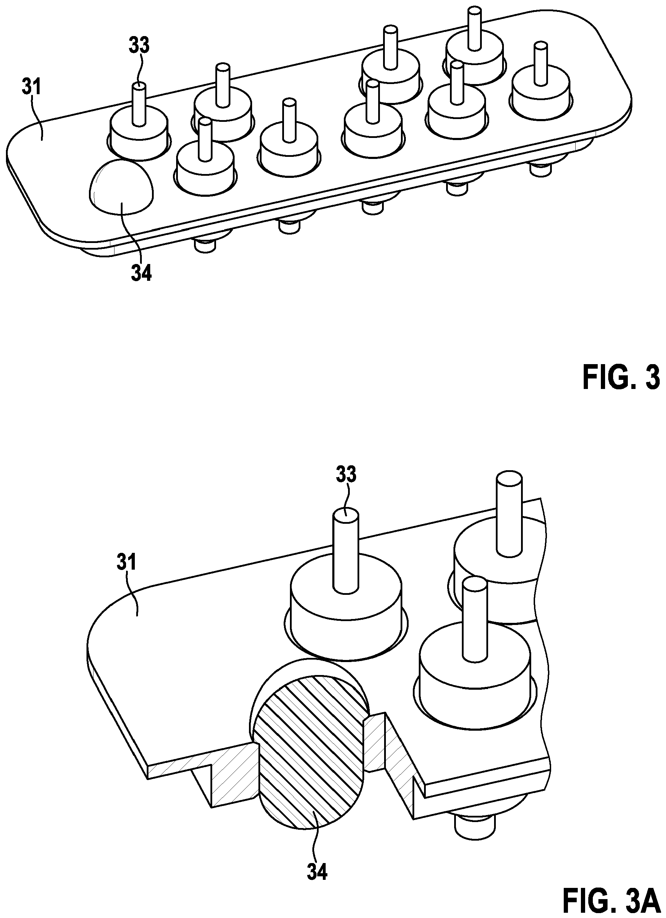

Fig. 3 zeigt, dass in einer ähnlichen Durchführung 31 mit einer Mehrzahl von Kontaktstiften 33 anstelle einer Glasschreibe ein einseitig konvexer Kunststoffkörper 34 als Lichtfenster mit Strahlformungseigenschaften eingebracht ist. Der Kunststoffkörper ist in die für ihn vorgesehene Bohrung eingepresst oder eingeklebt. Er kann auch beidseitig plan oder bikonvex oder auch bikonkav oder konvex/konkav geformt sein und somit in seiner geometrischen Konfiguration konkreten Anforderungen des jeweiligen Einsatzfalles angepasst sein.

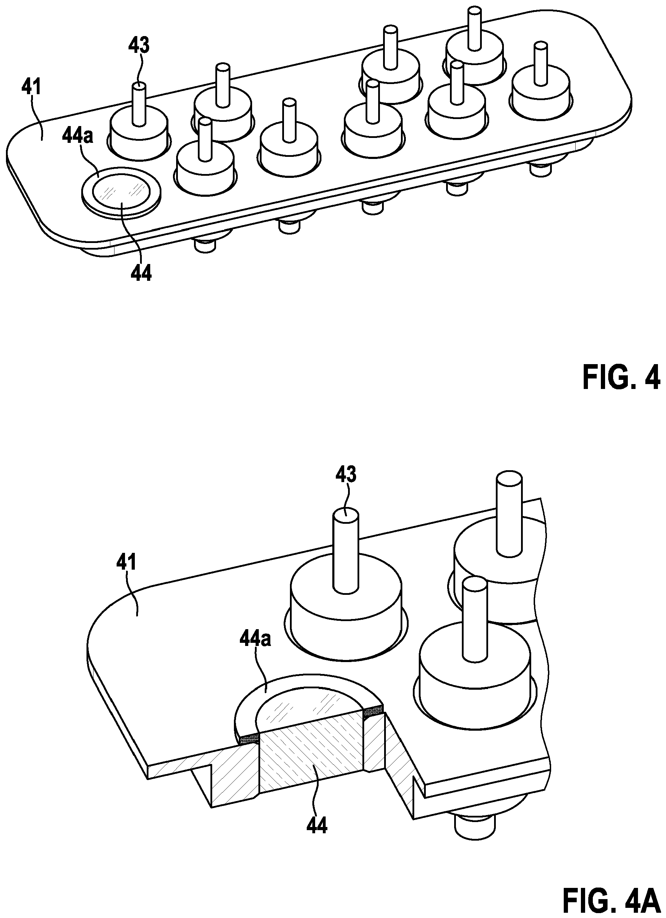

Fig. 4 zeigt als weiteres Ausführungsbeispiel eine wiederum zu den Durchführungen gemäß Fig. 2 und 3 ähnliche Durchführung 41 in der neben einer Mehrzahl von Kontaktstiften 43 eine licht- oder IR-durchlässige Scheibe 44 mit umlaufendem Klebstoffring 44a eingesiegelt ist. Die Scheibe wird bei einem Gerät, welches für eine Signalübertragung auf IR-Basis ausgebildet ist, aus einem für die IR-Strahlung der dabei eingesetzten Wellenlänge durchlässigen Material bestehen, also beispielsweise einem Kunststoff auf HDPE-Basis, einem Chalkogenidglas mit metallischer Dotierung o.ä.

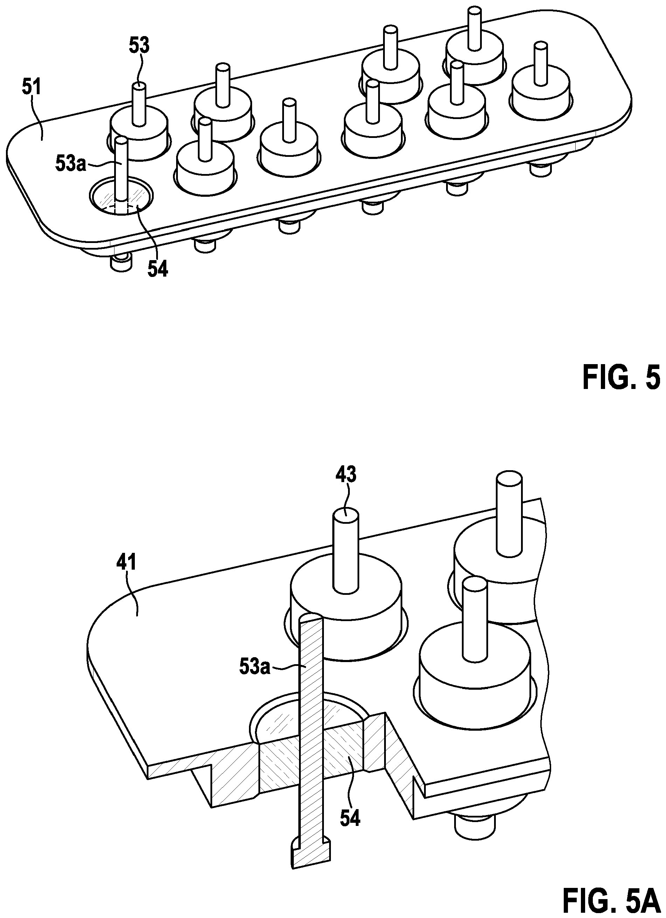

Fig. 5 und 5a zeigen als weiteres Beispiel eine Durchführung 51, in der neben normalen Anschlussstiften 53 ein Anschlussstift 53a vorgesehen ist, der sich durch das Zentrum einer Glasscheibe 54 erstreckt, die als Lichtfenster für die optische Signalübertragung dient und eine der weiter oben beschriebenen Ausführungen haben kann.

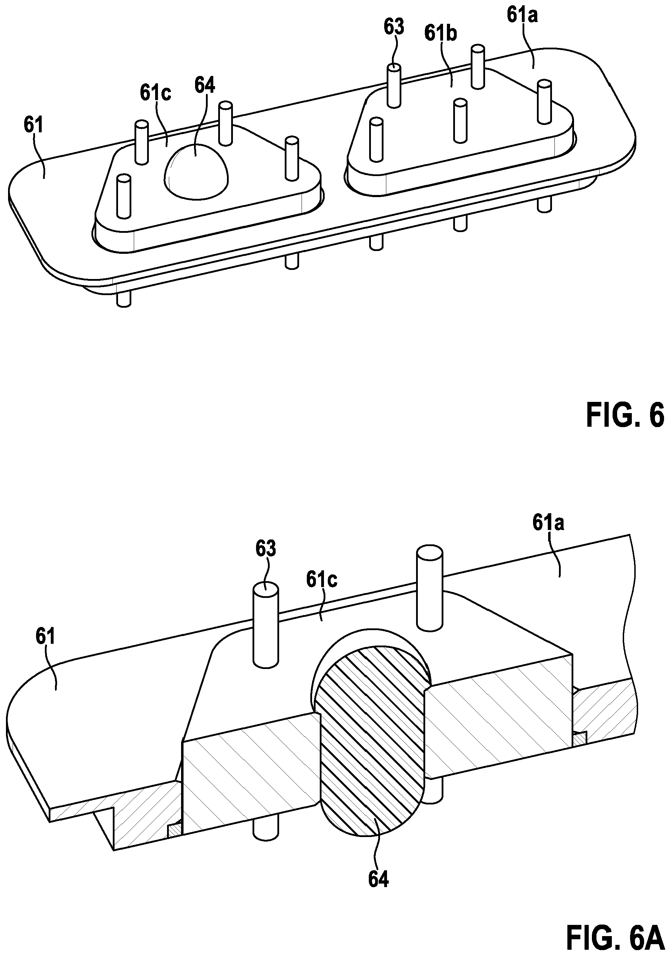

Fig. 6 und 6a zeigen als weiteres Beispiel eine etwas anders aufgebaute Durchführung 61, bei der in einem Durchführungs-Grundkörper 61a zwei Kontaktelement-Baugruppen 61b und 61c fixiert sind. Beide Kontaktelement-Baugruppen 61b, 61c enthalten jeweils eine Mehrzahl von Kontaktstiften 63, und die Kontaktelementbaugruppe 61c umfasst überdies ein Kunststoff-Lichtfenster 64 der in Fig. 3 gezeigten und weiter oben beschriebenen Art.

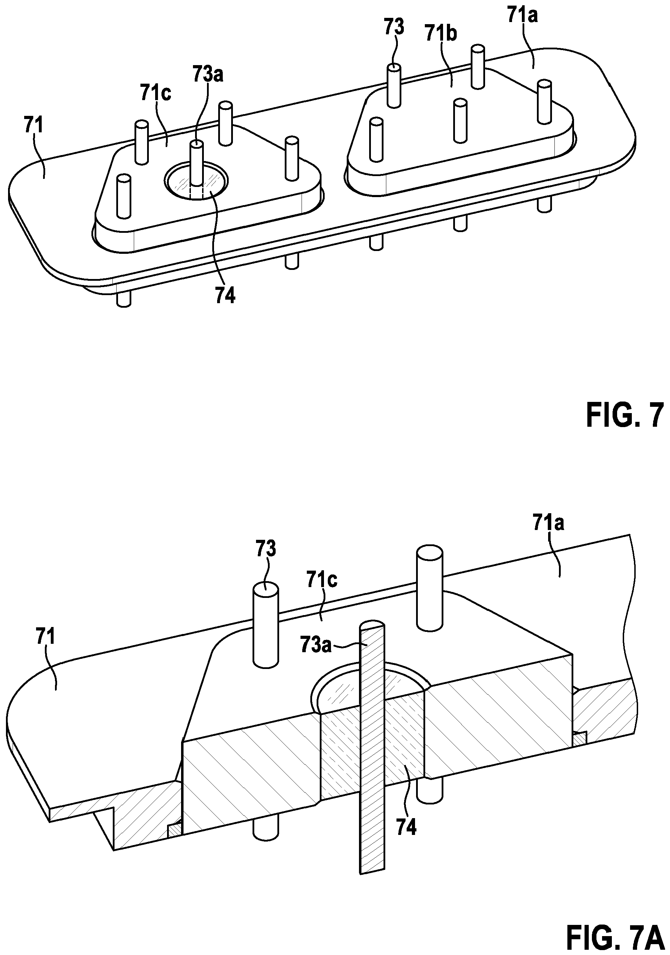

Fig. 7 und 7a schließlich zeigen eine zu Fig. 6 und 6a ähnliche Konfiguration einer Durchführung 71, bei der jedoch in einer der beiden Kontaktelement-Baugruppen 71b, 71c eine Lichtfenster/Anschlusspin-Kombination 74/73a vorgesehen ist, wie sie in Fig. 5 und 5a gezeigt und weiter oben beschrieben wurde.

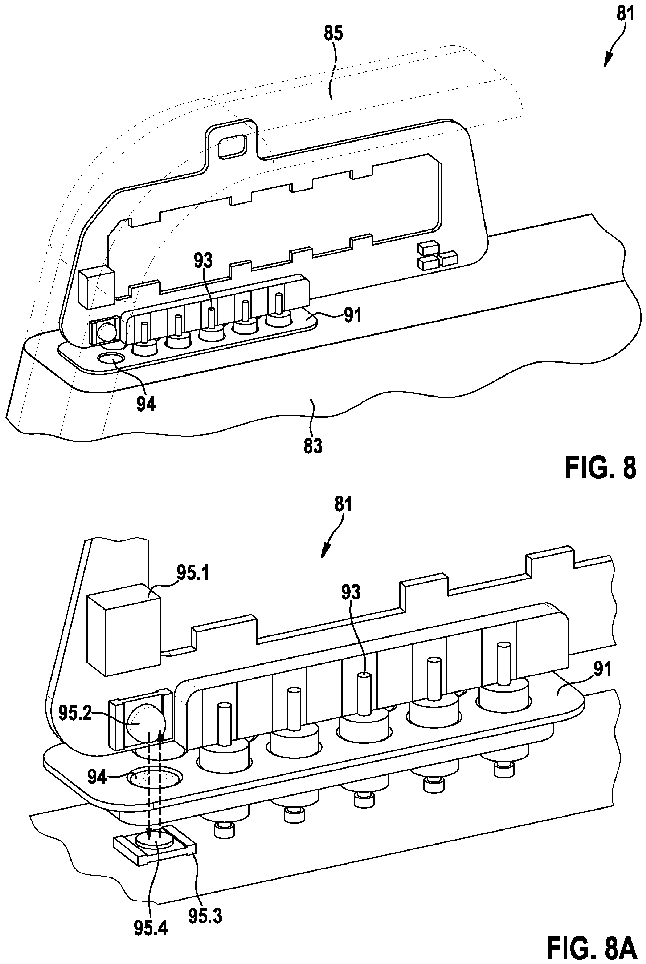

Fig. 8 und 8a zeigen eine perspektivische Teilansicht bzw. Detaildarstellung hierzu eines Herzschrittmachers 81 mit einem Gerätegehäuse 83 und einem Kopfteil (Header) 85, die mittels einer Durchführung 91 hermetisch dicht voneinander getrennt und mittels Anschlussstiften 93 und eines Lichtfensters 94 signalmäßig miteinander verbunden sind. Fig. 8a zeigt, dass dem Lichtfenster 94 Komponenten zur optischen Signalübertragung zugeordnet sind, und zwar im Kopfteil eine Codierungseinheit 95.1 und ein Sendeelement (beispielsweise eine LED) 95.2 und im Geräteinneren ein Auswertungs-IC 95.3 mit integriertem Lichtempfänger (Fotoelement) 95.4. Diese Darstellung ist lediglich als Prinzipskizze zu verstehen. Die Anordnung der Komponenten zum Kopfteil bzw. Gerätegehäuse kann auch umgekehrt sein, und im Übrigen ist die Realisierung von miniaturisierten optischen Signalübertragungsstrecken seit langem bekannt, und ihre Ausführung gehört zum fachmännischen Wissen.

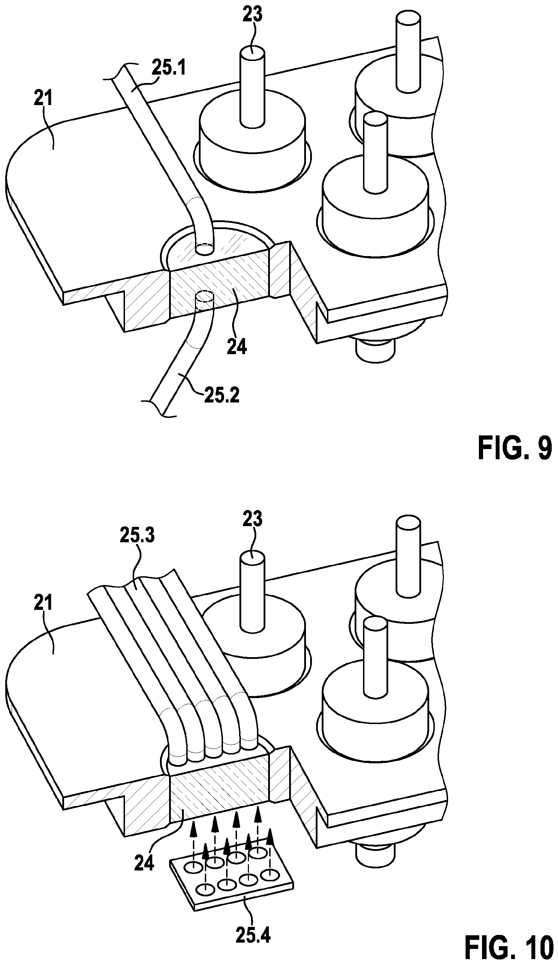

Fig. 9 und 10 zeigen als weitere Ausführungsbeispiele der Erfindung jeweils eine Durchführung 21, deren Aufbau grundsätzlich der Durchführung 21 nach Fig. 2 entspricht und mit in jener Figur benutzten Bezugsziffern bezeichnet ist. In Fig. 9 ist dem Licht- oder Infrarotfenster 24 auf beiden Seiten jeweils das Ende eines Lichtleiters 25.1 bzw. 25.2 zugeordnet. Die Lichtleiter 25.1, 25.2 können beispielsweise auf das Fenster 24' aufgeklebt sein; es kann aber auch ausreichen, sie einfach durch mechanische Fixierungsmittel stabil am Fenster 24 zu fixieren.

In Fig. 10 ist eine Variante dargestellt, bei der auf einer Seite des Fensters 24 ein Lichtleiterbündel 25.3 fixiert ist, während unter der gegenüberliegenden Seite des Fensters ein LED-Array 25.4 platziert ist, das eine der Anzahl der Lichtleiter im Lichtleiterbündel 25.3 entsprechende Anzahl von (nicht einzeln bezeichneten) LEDs umfasst. Mittels des LED-Arrays 25.4 und des Lichtleiterbündels 25.3 wird durch das einzelne Licht- oder Infrarotfenster 24 eine mehrkanalige optische Signalübertragung realisiert, die mehrere einzelne Anschlusselemente einer herkömmlichen Durchführung funktionsmäßig ersetzt.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Durchführung (11; 21; 31; 41; 51; 61; 71; 91) eines, insbesondere implantierbaren, medizinelektronischen Gerätes (1), welches ein Gerätegehäuse (3) aufweist, in dem elektronische und/oder elektrische Funktionseinheiten (7) untergebracht sind und das eine mit der Durchführung verschlossene Gehäuseöffnung hat, wobei die Durchführung einen Isolierkörper (61a, 71a), einen den Isolierkörper umfassenden und an der Gehäuseöffnung halternden Durchführungs-Flansch (12) und eine Mehrzahl von den Isolierkörper durchstoßenden Anschlusselementen (23, 24; 33, 34; 43, 44; 53, 53a, 54; 63, 64; 73, 73a, 74; 93, 94) zum externen Anschluss mindestens einer Funktionseinheit des Gerätes aufweist,
wobei mindestens ein Anschlusselement ein hermetisch dicht in den Isolierkörper oder direkt in den Durchführungs-Flansch eingefügtes Licht- oder Infrarotfenster (24; 34; 44; 54; 64; 74; 94) aufweist.

2. Durchführung nach Anspruch 1, wobei das Licht- oder Infrarotfenster ein in den Isolierkörper oder direkt in den Durchführungs-Flansch eingeschmolzenes oder eingeklebtes Glasfenster (24; 54; 74) ist.

3. Durchführung nach Anspruch 1, wobei das Licht- oder Infrarotfenster ein in den Isolierkörper oder direkt in den Durchführungs-Flansch eingespritztes, eingepresstes oder eingeklebtes Kunststofffenster (34; 64) ist.

4. Durchführung nach einem der vorangehenden Ansprüche, wobei das Licht- oder Infrarotfenster (24; 34; 44; 54; 64; 74; 94) als Kreisscheibe oder als einen zylindrischen Anschlussstift umgebender Kreisring konfiguriert ist.

5. Durchführung nach einem der vorangehenden Ansprüche, wobei der Isolierkörper (61 a; 71 a) aus Keramik besteht.

6. Durchführung nach einem der vorangehenden Ansprüche, wobei der Isolierkörper teilweise durch das Licht- oder Infrarotfenster gebildet ist.

7. Medizinelektronisches Gerät (1), insbesondere implantierbares Gerät, mit einer Durchführung (11; 21; 31; 41; 51; 61; 71; 91) nach einem der vorangehenden Ansprüche, insbesondere ausgebildet als Neurostimulator, Cochlearimplantat oder Retinaimplantat.

8. Gerät nach Anspruch 7, welches ein Kopfteil (5; 85) aufweist, wobei die Durchführung (11; 91) das Gerätegehäuse (3; 83) vom Kopfteil trennt.

9. Medizinelektronisches Gerät (1), insbesondere implantierbares Gerät, weiter insbesondere ausgebildet als Neurostimulator, Cochlearimplantat oder Retinaimplantat, welches ein Gerätegehäuse (3) aufweist, in dem elektronische und/oder elektrische Funktionseinheiten (7) untergebracht sind, wobei im Gerätegehäuse mindestens ein hermetisch dicht eingefügtes Licht- oder Infrarotfenster zur Realisierung eines externen Anschlusses mindestens einer der Funktionseinheiten des Gerätes über eine licht- oder Infrarotoptische Signalverbindung vorgesehen ist.

10. Gerät nach einem der Ansprüche 7 bis 9, wobei dem oder mindestens einem Licht- oder Infrarotfenster (24) im Gerätegehäuse und/oder im Kopfteil mindestens ein Lichtleiterabschnitt (25.1, 25.2; 25.3) zugeordnet ist, über den eine optische Signalübertragung durch das Fenster hindurch realisiert wird.

11. Gerät nach Anspruch 10, wobei dem oder mindestens einem Licht- oder Infrarotfenster (24) eine Mehrzahl von Lichtleiterabschnitten (25.3) zugeordnet ist, derart, dass durch das Fenster hindurch eine mehrkanalige optische Signalübertragung realisiert wird.

12. Gerät nach einem der Ansprüche 7 bis 11, wobei ein freies Ende des Lichtleiters oder der Lichtleiter (25.1, 25.2; 25.3) direkt an eine Oberfläche des Licht- oder Infrarotfensters (24) angefügt ist.

13. Gerät nach einem der Ansprüche 7 bis 12, wobei dem oder mindestens einem Licht- oder Infrarotfenster (94; 24") mindestens eine LED (95.2; 25.4) oder ein Halbleiterlaserelement zugeordnet ist, über die/das eine optische Signalübertragung durch das Fenster hindurch realisiert wird.

14. Gerät nach einem der Ansprüche 7 bis 13, wobei in der Durchführung (51; 71) ein einen zylindrischen Anschlussstift (53a, 73a) umgebendes kreisringförmiges Licht- oder Infrarotfenster (54; 74) vorgesehen und der vom Fenster umgebene Anschlussstift mit einem Versorgungsstromleiter zur Energieversorgung mindestens einer Funktionseinheit (7) des Gerätes (1) verbunden ist.
